# EUROPEAN PATENT SPECIFICATION

(11) **EP 2 020 245 B1**
(45) Date of publication and mention of the grant of the patent: **30.11.2011**
(21) Application number: 07425491.3
(22) Date of filing: 31.07.2007
(51) Int. Cl.: A61L 2/04, A61F 9/00, A61M 5/00, A61K 9/00

(54) **Method for making a device for ophthalmic treatments**
Methode zum Herstellen einer Vorrichtung für ophthalmische Behandlungen
Méthode pour la réalisation d'un dispositif pour traitements ophtalmiques

(43) Date of publication of application: 04.02.2009
(73) Proprietor: AL.CHI.MI.A. S.r.l., 35020 Ponte S. Nicolò (Padova) (IT)
(72) Inventor: Beccaro, Mauro, 35010 Cadoneghe (Padova) (IT); Bettini, Enrico, 30032 Fiesso d'Artico (Venezia) (IT); Signori, Paolo, 37131 Verona (IT)
(74) Representative: Ponchiroli, Simone

(56) References cited:
- WO-A-98/45191
- US-A- 4 628 969
- US-A- 5 037 384
- US-A- 5 336 175
- US-A1- 2004 078 993
- US-A1- 2004 099 994
- US-A1- 2004 228 759
- US-B1- 6 599 280

## Description

The present invention relates to a device for ophthalmic treatments and a method for making said device.

In particular, the present invention relates to a method for making a new device which can be used in the surgical treatment of ophthalmic pathologies, in particular for injecting a liquid compound into the eyeball.

As described for example in patents US 5,037,384, US 5,336,175 and US 5,792,099, some ophthalmic pathologies, such as a detached retina, are treated surgically by injecting a liquid which constitutes a surgical internal tamponade agent into the eye. In particular, in the case of treatment of a detached retina, a liquid, selected from amongst the family of perfluorocarbons, is injected into the eyeball. Thanks to its high surface tension, said liquid, completely or partly substituting the vitreous humour present in the eyeball, generates a thrust on the part of the retina detached and curled, smoothing it out and keeping it adherent to the inner wall of the eyeball, allowing its subsequent reattachment.

At present, said liquid compounds are usually inserted in the posterior chamber using a normal syringe which, after being taken out of a respective package (designed to keep the syringe sterile), is initially connected to a needle. The surgeon then uses the syringe to make a hole in a rubber cap of a container for the sterile liquid compound and draws out the desired quantity of the compound.

At this point, the needle is separated from the syringe and substituted with a cannula suitable for the intraocular injection.

However, said method has significant disadvantages. A first disadvantage is the fact that the manual operations for connection and substitution of the needle with the cannula, like drawing out the liquid, involve a significant increase in the risks of microbiological contamination of the entire device. Despite the conditions for controlling microbiological contamination in operating theatres, the presence of machinery, equipment and medical personnel significantly increases bacterial contamination in the operating theatre.

To overcome said disadvantage, the syringes often have a filter to decontaminate the liquid supplied through the cannula.

Secondly, use of such a method is not very practical given that it requires many operations for preparing the syringe, drawing the liquid, substituting the needle, etc.

A different solution is described in patent US 5,037,384 which involves the use of a container for a liquid perfluorocarbon, at one end having a dispenser cannula intercepted by a specific valve and, at the other end a cap through which, by making a hole, it is possible to insert a needle connected to a pressurised air source to allow expulsion of the liquid from the container through the cannula. Said container can therefore be used in place of common syringes.

However, that solution is also not without the risk of contamination, since on each occasion it requires insertion in the container of a needle and air coming from the outside.

Moreover, the cost of the device is also relatively high due to the presence of the on - off valve. Also, the use of said device necessitates the availability of a source of compressed air.

It is also known from WO 98/45191 a unit dose, gas-filled syringe which is filled with a gas and packaged in a gas barrier material prior to use to increase shelf-life, that is, to minimize gas leakage and dilution of the contents of the syringe. The syringe is filled with a selected gas and sealed inside a container made from a high gas barrier material. The container is also filled with the selected gas. The container material is selected to have a gas transmission rate sufficient to prevent the selected gas from diffusing out of the container into the atmosphere. The pressure of the gas in the container is greater than the atmospheric pressure to prevent the atmospheric contaminants from entering the container and syringe.

Document US 2004/078993 discloses a method for sterilising a syringe which has a shiftable plunger that defines a compartment filled with a fluid. The container is confined in a pressurizable chamber and heated so as to change a pressure in the compartment of the container. The pressure in the compartment of the container is monitored and an output corresponding thereto is generated. Pressure in the chamber around the container is continuously varied so as to be generally equal to the instantaneous monitored pressure in the compartment of the container. In this manner the plunger is not moved by thermal expansion or contraction of the fluid.

Finally, document US 2004/228759 discloses a process for operating a packaging transport system in which objects packed in packaging which is bacteria-impermeable but gas-permeable are sterilized, where after the packaging, possibly after removal of some packaging parts, is sterilized again on its outer side in a sterilization chamber which acts as a transfer lock, and then it is guided into a sterile clean room. The objects involved may, in particular, be syringes having injecting needles, which are filled and sealed in a filling device located in the clean room. Sterilization in the sterilization chamber is accomplished by abruptly applying a vapor mix consisting of water steam and hydrogen peroxide vapor as a condensate layer on the outer side of the transport containers, and immediately removing the condensate layer and the uncondensed vapor mix from the sterilization chamber before any significant amount of hydrogen peroxide is deposited inside the transport containers.

In this context, the main aim of the present invention is to provide method for making a device for use in ophthalmic surgery which is free of the disadvantages indicated above.

In particular, the aim of the present invention is to provide method for making a device for ophthalmic treatments which is easy and practical to use.

The present invention also has for an aim to provide method for making a device for ophthalmic treatments able to minimise the risks of microbiological contamination.

The present invention has then for technical purpose to provide a method for making such a device for ophthalmic treatments.

The technical purpose indicated and the aims specified are substantially achieved by a method for making such a device comprising the technical features described in one or more of the claims herein.

Further features and advantages of the present invention are more apparent in the detailed description below, with reference to a preferred, non-limiting, embodiment of a device for ophthalmic treatments, illustrated in the accompanying drawings, in which:
- Figure 1 is a perspective view of an embodiment of a device for ophthalmic treatments in accordance with the present invention; and
- Figure 2 is a perspective view of the device of Figure 1 with some parts cut away (package and Luer connector) to better illustrate others; and
- Figure 3 is a cross-section of a detail of the device of Figure 1.

With reference to the accompanying drawings, the numeral 1 denotes as a whole a device for ophthalmic treatments made in accordance with the present invention.

As already indicated, the device 1 is advantageously applied for the treatment of ophthalmic pathologies of various types, in particular detached retinas.

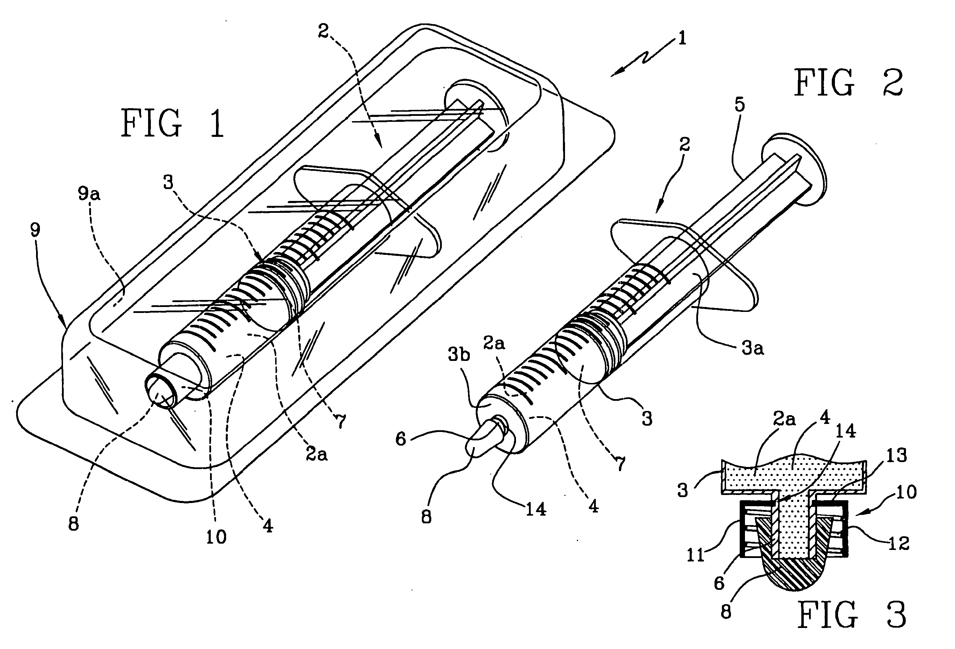

The device 1 in general comprises a package 9 which forms a housing chamber 9a inside, and a syringe 2 inserted in the housing chamber 9a and filled with a sterile liquid 4, of the type which can be injected into an eyeball (in the accompanying Figures 1 and 2 the liquid 4 is not visible because it is considered transparent). The syringe may also be sterile.

Depending on the applications, the package 9 may have different forms. For example, it may consist of a blister pack as illustrated, a bag (usually, in the known way, consisting of a film of plastic material joined to a sheet of medical paper), a package of another type, a rigid container, etc. Moreover, advantageously, the package 9 is sealed and, inside the housing chamber 9a, and around the syringe 2, there is a certain quantity of sterile air.

The syringe 2 in turn comprises a hollow tubular body 3 having an open first end 3a and an open second end 3b, and a stopper 7 partly slidably inserted in a sealed fashion in said hollow tubular body 3 through the first end 3a.

The second end 3b is closed by a cap 8.

Depending on the embodiments, the stopper 7, which forms a seal against the inner wall of the tubular body 3, may be connected or may be able to connect to a plunger 5 which can be partly inserted in the tubular body 3 through the first end 3a. If the plunger 5 is not connected to the stopper 7, it is in any case preferably inserted in the package 9 and can usually be connected to the stopper 7 by screwing.

The liquid 4 is contained in a containment chamber 2a delimited, in the tubular body 3, by the stopper 7 and by the cap 8. In some preferred embodiments, the quantity of liquid 4 inserted in the syringe 2 is equal to a single dose, so that the entire device 1 is a disposable device.

In the embodiment illustrated in the accompanying drawings, rigidly connected at the second end 3b of the tubular body 3 there is a projecting tip 6, to which, in practice, the cannula (not illustrated in the accompanying drawings) is connected for supplying the liquid. In particular, in the embodiment illustrated in the accompanying drawings, to allow connection of the cannula, a standard type Luer connector 10 is attached to the tip 6. The Luer connector consists of a cylindrical element 11 with internal threading 12 (to which the cannula can be screwed) mounted around the tip 6, with which it is made integral by fitting a disk-shaped portion 13 of it with a hole made in it into a circular channel 14 made on the tip 6 (Figure 3). Advantageously, the tip 6 is closed by the cap 8 which can be removed at the moment of using the syringe 2 to allow connection of the cannula to the tip 6.

In the preferred embodiment, given the chemical composition of the liquids preferably used, the tubular body 3 is made of glass, whilst both the stopper 7 and the cap 8 are made of rubber (preferably a butyl - based mixture, such as, in particular chlorobutyl and bromobutyl) and are coated with a polymeric film which is inert relative to the liquid 4, flexible and preferably consists of a fluorinated polymer.

The liquid 4 in the syringe 2 is usually a product which is liquid at atmospheric temperature and pressure, and is selected from amongst the family of perfluorocarbons, advantageously used in ophthalmic pathologies and in particular in the treatment of detached retinas (internal tamponade agents for retinal surgery).

In particular, the liquid 4 preferably comprises a compound selected from amongst the group comprising: perfluorohexane (C₆F₁₄), perfluorohydrophenanthrene (C₁₄F₂₄), perfluorooctane (C₈F₁₈), perfluoro-n-octane (C₈F₁₈), perfluorodecalin (C₁₀F₁₈), perfluoromethyldecalin (C₁₁F₂₀).

Advantageously, the package 9 is sealed, using known packaging methods, to keep the housing chamber 9a sterile and avoid any bacterial contamination of the syringe 2.

The device 1 described above is therefore a product ready for use in which it is sufficient to remove the cap 8 and insert the cannula to be able to supply the liquid 4 to be injected. It should be noticed that, although the cannula is not illustrated in the accompanying drawings, it may advantageously be inserted in the package 9 containing the syringe 2.

The device 1 described above mainly in a structural sense may be made using the method according to the present invention, described below.

The method in general involves the following operating steps, in the order indicated:
- a step of preparing the syringe 2 forming the containment chamber 2a on the inside;
- a step of inserting in the containment chamber 2a a liquid 4 to be injected into an eyeball;
- and a step of sterilising the syringe 2 filled with the liquid 4 by means of heat treatment, so as to simultaneously treat both the liquid 4 and the syringe 2.

However, the heat treatment step must be carried out while keeping the stopper 7 stationary relative to the cylindrical body 3.

Many of the liquids used have boiling temperatures lower than or just above the heat treatment temperatures, as well as a vapour pressure that significantly increases with an increase in the temperature.

Consequently, during the heat treatment step, inside the containment chamber 2a a relatively high pressure develops which in itself would be sufficient to cause the stopper 7 to move towards the first end of the cylindrical body, or even beyond it.

Since any movement, even minimal, of the stopper 7 may cause a risk of contamination and the product escaping, the stopper must be prevented from moving during the heat treatment.

Although this is possible by means of mechanical blocking, in the preferred embodiment, for the entire duration of the heat treatment, an overpressure atmosphere is created on the outside of the syringe 2, counterbalancing the pressure present in the containment chamber 2a.

The sterilising heat treatment can usually be carried out using an autoclave (of the known type and therefore not described and illustrated in any more detail).

In particular, the filled syringe 2 is inserted in the autoclave and heated to a predetermined treatment temperature (for example corresponding to the sterilising temperature).

The heating step is usually carried out for a heating time less than or equal to 30 minutes during which the predetermined treatment temperature is reached which, in the case of sterilisation, is advantageously between 121°C and 124°C.

Then, the predetermined treatment temperature is maintained for a treatment time, preferably between 15 and 30 minutes.

After the step during which the heat treatment temperature was maintained, the syringe 2 filled with the liquid 4 is cooled to a cooling temperature.

The cooling step is preferably carried out for a cooling time less than or equal to 45 minutes during which the cooling temperature is reached, advantageously between 50°C and 60°C.

As already indicated, in the preferred embodiment, during the step of heating, maintaining the heat treatment temperature and cooling, inside the autoclave a predetermined overpressure is maintained relative to the theoretical vapour pressure. Said predetermined overpressure is preferably between 0.5 and 2 bar, in such a way that during the entire treatment, the pressure is between 1.5 and 3.8 bar (the highest pressure peak is reached during maintenance of the heat treatment temperature).

This is obtained for example by means of an air - steam sterilisation process in balanced counterpressure, during the entire duration of which inside the autoclave a predetermined quantity of air is maintained sufficient to create said overpressure. Therefore, when the syringe is placed in the autoclave, rather than creating a vacuum, the air already present is left and during the subsequent treatment, in addition to the steam, if necessary additional air is blown into the autoclave.

To obtain the device 1 described above, a step is also needed of packaging the syringe 2 in the package 9.

Depending on requirements, packaging can be done either after the heat treatment step, working in an environment with a sterile atmosphere, or before the heat treatment step. In the latter case, the syringe 2 filled with the liquid 4, is subjected to the heat treatment already packaged in the package. Therefore, even the atmosphere contained in the housing chamber 9a formed by the package 9 is sterilised by the heat treatment.

Moreover, in the latter case if the package is rigid it may in itself contribute to the mechanical blocking of the plunger 5 (if the latter is connected to the stopper 7), whilst if it is squeezable the pressure in the autoclave is the same as that in the housing chamber 9a.

Preparation of the liquid 4 may in turn comprise a step of weighing the raw materials of which the liquid 4 consists and a step of sterilising filtration of the liquid 4, again all advantageously carried out in sterile conditions. The present invention brings important advantages.

Advantageously, the device disclosed is ready for use during a surgical operation. Since the device is already sterilised and ready-filled, the medical personnel only have to take the syringe out of the package and apply the cannula to the tip in place of the cap.

Consequently, the risks of microbiological contamination are minimised, since the device does not have to be subjected to laborious processes for filling and substitution of the needle with the cannula.

Moreover, it should be noticed that use of the device is practical and immediate, with the consequent advantage in terms of the duration of the entire surgical operation.

In addition, thanks to the fact that they are disposable devices, it is possible to avoid the presence of vials to be reused several times, as well as the need to apply a sterilising filter to the syringe.

Thanks to the production method described above, it has been possible to make the present device despite the critical physical properties of the liquids used.

## Claims

1. A method for making a device for ophthalmic treatments comprising a package (9) forming a housing chamber (9a) inside it, and a syringe (2) inserted in the housing chamber (9a), the syringe (2) being filled with a sterile liquid which can be injected into an eyeball, selected from amongst perfluorocarbons, **characterised in that** it comprises the steps of:
- preparing a syringe (2) forming a containment chamber (2a) on the inside;
- inserting in the containment chamber (2a) the sterile liquid (4); and
- sterilising the syringe (2) filled with the liquid (4) by means of heat treatment, the sterilising step being carried out while preventing variations in the volume of the containment chamber (2a); and of packaging the syringe (2) in a package (9) designed to protect it from any contamination.

2. The method according to the foregoing claim, **characterised in that** variations in the volume of the containment chamber (2a) are prevented by creating an overpressure atmosphere outside the syringe.

3. The method according to claim 1 or 2, **characterised in that** the step of sterilising by heat treatment comprises the steps of:
- heating the syringe (2) filled with the liquid (4) to a predetermined treatment temperature; then
- keeping the syringe (2) at the treatment temperature for a predetermined treatment time; and
- cooling the syringe (2) filled with the liquid (4) to a cooling temperature.

4. The method according to claim 3, **characterised in that** the predetermined treatment temperature corresponds to a sterilising temperature for the liquid.

5. The method according to the foregoing claim, **characterised in that** the treatment temperature is between 121°C and 124°C.

6. The method according to any of the claims from 1 to 5, **characterised in that** the treatment time is between 15 and 30 minutes.

7. The method according to any of the claims from 2 to 6, **characterised in that** the heating step is carried out for a heating time less than or equal to 30 minutes.

8. The method according to any of the claims from 2 to 7, **characterised in that** the cooling step is carried out for a cooling time less than or equal to 45 minutes.

9. The method according to any of the claims from 2 to 7, **characterised in that** the cooling temperature is between 50°C and 60°C.

10. The method according to any of the claims from 1 to 8, **characterised in that** the step of sterilising by heat treatment is carried out in an autoclave.

11. The method according to claims 2 and 10, **characterised in that** to create the overpressure atmosphere, a predetermined overpressure is created relative to the theoretical vapour pressure in the autoclave.

12. The method according to the foregoing claim, **characterised in that** the predetermined overpressure relative to the theoretical vapour pressure in the autoclave is between 0.5 and 2 bar.

13. The method according to any of the claims from 1 to 12, **characterised in that** it also comprises a step of packaging the syringe (2) in a package (9) designed to protect it from any contamination.

14. The method according to the foregoing claim, **characterised in that** the packaging step is carried out after the step of cooling and in a sterile atmosphere.

15. The method according to claim 13, **characterised in that** the packaging step is carried out after the step of inserting the liquid (4) in the syringe (2) and before the step of sterilising by heat treatment which therefore also affects the package.

16. The method according to any of the claims from 1 to 15, **characterised in that** it also comprises the step of eliminating the presence of air in the syringe (2) after the step of introducing the liquid (4).

17. The method according to any of the claims from 1 to 16, **characterised in that** it also comprises the step of eliminating the presence of air in the syringe (2) after the step of introducing the liquid (4).

## Patentansprüche

1. Methode zum Herstellen einer Vorrichtung für ophtalmische Behandlungen, enthaltend eine Konfektion (9), die in ihrem Inneren eine Aufnahmekammer (9a) bildet, sowie eine Spritze (2), eingesetzt in die Aufnahmekammer (9a), wobei die Spritze (2) mit einer sterilen Flüssigkeit gefüllt ist, welche in den Augapfel eingespritzt werden kann, ausgewählt aus Perfluorkohlenstoffen, **dadurch gekennzeichnet, dass** sie folgende Phasen enthält:
- Vorbereitung einer in ihrem Inneren eine Füllkammer (2a) bildenden Spritze (2);
- Einfüllen der sterilen Flüssigkeit (4) in die Füllkammer (2a); und
- Sterilisierung der mit der Flüssigkeit (4) gefüllten Spritze (2) durch eine Wärmebehandlung, wobei die Sterilisierphase ausgeführt wird unter Verhinderung von Veränderungen im Volumen der Füllkammer (2a); und das Verpacken der Spritze (2) in einer Konfektion (9), dazu bestimmt, sie vor jeder Kontaminierung zu schützen.

2. Methode nach dem vorstehenden Patentanspruch, **dadurch gekennzeichnet, dass** die Veränderungen im Volumen der Füllkammer (2a) durch die Erzeugung eines Überdruckes ausserhalb der Spritze verhindert werden.

3. Methode nach Patentanspruch 1 oder 2, **dadurch gekennzeichnet, dass** die Phase der Sterilisierung durch Wärmebehandlung die folgenden Schritte enthält:
- Erhitzen der mit der Flüssigkeit (4) gefüllten Spritze (2) bis aus eine vorbestimmte Behandlungstemperatur; dann
- Halten der Spritze (2) auf der Behandlungstemperatur für eine vorgegebene Behandlungsdauer; und
- Abkühlen der mit der Flüssigkeit (4) gefüllten Spritze (2) bis auf eine Kühltemperatur.

4. Methode nach Patentanspruch 3, **dadurch gekennzeichnet, dass** die vorbestimmte Behandlungstemperatur einer Sterilisiertemperatur für die Flüssigkeit entspricht.

5. Methode nach dem vorstehenden Patentanspruch, **dadurch gekennzeichnet, dass** die Behandlungstemperatur zwischen 121°C und 124°C liegt.

6. Methode nach einem jeden der vorstehenden Patentansprüche von 1 bis 5, **dadurch gekennzeichnet, dass** die Behandlungsdauer zwischen 15 und 30 Minuten beträgt.

7. Methode nach einem jeden der vorstehenden Patentansprüche von 2 bis 6, **dadurch gekennzeichnet, dass** die Phase des Erwärmens während einer Erwärmungsdauer unter oder gleich 30 Minuten durchgeführt wird.

8. Methode nach einem jeden der vorstehenden Patentansprüche von 2 bis 7, **dadurch gekennzeichnet, dass** die Phase des Abkühlens während einer Abkühldauer unter oder gleich 45 Minuten durchgeführt wird.

9. Methode nach einem jeden der vorstehenden Patentansprüche von 2 bis 7, **dadurch gekennzeichnet, dass** die Kühltemperatur zwischen 50°C und 60°C liegt.

10. Methode nach einem jeden der vorstehenden Patentansprüche von 1 bis 8, **dadurch gekennzeichnet, dass** die Sterilisierphase durch Wärmebehandlung in einem Autoklav erfolgt.

11. Methode nach den Patentansprüchen von 2 und 10, **dadurch gekennzeichnet, dass** zum Erzeugen des atmosphärischen Überdruckes ein bestimmter Überdruck im Verhältnis zu dem theoretischen Dampfdruck im Autoklav erzeugt wird.

12. Methode nach dem vorstehenden Patentanspruch, **dadurch gekennzeichnet, dass** der bestimmte Überdruck im Verhältnis zu dem theoretischen Dampfdruck in dem Autoklav zwischen 0,5 und 2 bar beträgt.

13. Methode nach einem jeden der vorstehenden Patentansprüche von 1 bis 12, **dadurch gekennzeichnet, dass** sie ebenfalls eine Phase des Verpackens der Spritze (2) in einer Konfektion (9) enthält, dazu bestimmt, die Spritze vor jeder Kontamination zu schützen.

14. Methode nach dem vorstehenden Patentanspruch, **dadurch gekennzeichnet, dass** die Phase des Verpackens nach der Kühlphase ausgeführt wird und in einer sterilen Atmosphäre erfolgt.

15. Methode nach Patentanspruch 13, **dadurch gekennzeichnet, dass** die Phase des Verpackens nach der Phase des Einfüllens der Flüssigkeit (4) in die Spritze (2) und vor der Phase der Sterilisierung durch Wärmebehandlung ausgeführt wird, welche daher auch die Verpackung einbezieht.

16. Methode nach einem jeden der vorstehenden Patentansprüche von 1 bis 15, **dadurch gekennzeichnet, dass** sie ebenfalls eine Phase des Entfernens von in der Spritze (2) vorhandener Luft enthält, und zwar nach der Phase des Einfüllens der Flüssigkeit (4).

17. Methode nach einem jeden der vorstehenden Patentansprüche von 1 bis 16, **dadurch gekennzeichnet, dass** sie ebenfalls eine Phase des Entfernens von in der Spritze (2) vorhandener Luft enthält, und zwar nach der Phase des Einfüllens der Flüssigkeit (4).

## Revendications

1. Une méthode pour la réalisation d'un dispositif pour traitements ophtalmiques comprenant un emballage (9) formant intérieurement une chambre de logement (9a), et une seringue (2) placée dans la chambre de logement (9a), la seringue (2) étant remplie d'un liquide stérile qui peut être injecté dans un globe oculaire, choisi parmi les perfluorocarbones, **caractérisée en ce qu'**elle comprend les phases consistant à :
- préparer une seringue (2) définissant intérieurement une chambre - contenant (2a) ;
- introduire dans ladite chambre - contenant (2a) le liquide stérile (4)
et
- stériliser la seringue (2) remplie de liquide (4) par le biais d'un traitement thermique, la phase de stérilisation étant effectuée en empêchant toutes variations du volume de la chambre - contenant (2a) ; et
- emballer la seringue (2) dans un emballage (9) destiné à la protéger de toute contamination.

2. La méthode selon la revendication précédente, **caractérisée en ce que** les variations du volume de la chambre - contenant (2a) sont empêchées en créant une atmosphère en surpression à l'extérieur de la seringue.

3. La méthode selon la revendication 1 ou 2, **caractérisée en ce que** la phase de stérilisation par traitement thermique comprend les phases consistant à :
- chauffer la seringue (2) remplie de liquide (4) jusqu'à une température de traitement prédéfinie ; puis
- maintenir la seringue (2) à la température de traitement pendant un temps de traitement prédéfini ; et
- refroidir la seringue (2) remplie de liquide (4) jusqu'à une température de refroidissement.

4. La méthode selon la revendication 3, **caractérisée en ce que** la température de traitement prédéfinie correspond à une température de stérilisation du liquide.

5. La méthode selon la revendication précédente, **caractérisée en ce que** la température de traitement est comprise entre 121°C et 124°C.

6. La méthode selon l'une quelconque des revendications de 1 à 5, **caractérisée en ce que** le temps de traitement est compris entre 15 et 30 minutes.

7. La méthode selon l'une quelconque des revendications de 2 à 6, **caractérisée en ce que** la phase de chauffage est effectuée pendant un temps de chauffage qui est inférieur ou égal à 30 minutes.

8. La méthode selon l'une quelconque des revendications de 2 à 7, **caractérisée en ce que** la phase de refroidissement est effectuée pendant un temps de refroidissement qui est inférieur ou égal à 45 minutes.

9. La méthode selon l'une quelconque des revendications de 2 à 7, **caractérisée en ce que** la température de refroidissement est comprise entre 50°C et 60°C.

10. La méthode selon l'une quelconque des revendications de 1 à 8, **caractérisée en ce que** la phase de stérilisation par traitement thermique est effectuée dans un autoclave.

11. La méthode selon les revendications 2 et 10, **caractérisée en ce que** pour créer l'atmosphère en surpression, une surpression prédéfinie est créée par rapport à la pression de vapeur théorique présente dans l'autoclave.

12. La méthode selon la revendication précédente, **caractérisée en ce que** la surpression prédéfinie par rapport à la pression de vapeur théorique présente dans l'autoclave est comprise entre 0,5 et 2 bars.

13. La méthode selon l'une quelconque des revendications de 1 à 12, **caractérisée en ce qu'**elle comprend aussi une phase consistant à emballer la seringue (2) dans un emballage (9) destiné à la protéger de toute contamination.

14. La méthode selon la revendication précédente, **caractérisée en ce que** la phase d'emballage est effectuée après la phase de refroidissement et dans une atmosphère stérile.

15. La méthode selon la revendication 13, **caractérisée en ce que** la phase d'emballage est effectuée après la phase d'introduction du liquide (4) dans la seringue (2) et avant la phase de stérilisation par traitement thermique qui concerne donc aussi l'emballage.

16. La méthode selon l'une quelconque des revendications de 1 à 15, **caractérisée en ce qu'**elle comprend aussi la phase consistant à éliminer la présence d'air dans la seringue (2) après la phase d'introduction du liquide (4).

17. La méthode selon l'une quelconque des revendications de 1 à 16, **caractérisée en ce qu'**elle comprend aussi la phase consistant à éliminer la présence d'air dans la seringue (2) après la phase d'introduction du liquide (4).
